# EUROPEAN PATENT APPLICATION

(11) **EP 3 932 395 A1**
(43) Date of publication of application: **05.01.2022**
(21) Application number: 20183965.1
(22) Date of filing: 03.07.2020
(51) Int. Cl.: A61K 9/14, A61K 31/423, A61K 47/10, A61K 47/12, A61K 47/14, A61P 7/10, A61P 17/04, A61P 17/08, A61P 17/14, A61P 17/16, A61P 25/00, A61P 29/00, A61P 35/00, A61P 37/08

(54) **STABLE EMULSION CONTAINING AN ACTIVE PHARMACEUTICAL INGREDIENT BASED ON N(3-METHYL-2-OXO-2,3-DIHYDROBENZO[D]OXAZOL-5-YL)DODECANAMIDE, PRODUCTION AND USE**

(71) Applicant: Dr. August Wolff GmbH & Co. KG Arzneimittel, 33611 Bielefeld (DE)
(72) Inventor: Christians, Thorsten, 33611 Bielefeld (DE); Knie, Ulrich, 32105 Bad Salzufen (DE)
(74) Representative: Ter Meer Steinmeister & Partner

(57) **Abstract**

The present invention relates to an oil-in-water emulsion containing N-(3-methyl-2-oxo-2,3-dihydrobenzo[d]oxazol-5-yl)dodecanamide or a derivative, solvate, hydrate, or prodrug thereof or a pharmaceutically acceptable salt thereof, and a specific emulsifier system. Furthermore, the present invention relates to such an emulsion for use as medicament, in particular for use in treating and/or preventing inflammation, irritation, itching, pruritus, pain, oedema, pro-allergic or allergic conditions in a patient. Usually, the medicament is a dermatological formulation which is topically applied to the skin or mucosa of a mammal. The present invention also relates to the non-therapeutic (cosmetic) use of the oil-in-water emulsion for treating cosmetic skin conditions and to a method for producing the oil-in-water emulsion.

## Description

The present invention relates to an oil-in-water emulsion containing N-(3-methyl-2-oxo-2,3-dihydrobenzo[d]oxazol-5-yl)dodecanamide or a derivative, solvate, hydrate, or prodrug thereof or a pharmaceutically acceptable salt thereof, and a specific emulsifier system. Furthermore, the present invention relates to such an emulsion for use as medicament, in particular for use in treating and/or preventing inflammation, irritation, itching, pruritus, pain, oedema, pro-allergic or allergic conditions in a patient. Usually, the medicament is a dermatological formulation which is topically applied to the skin or mucosa of a mammal. The present invention also relates to the non-therapeutic (cosmetic) use of the oil-in-water emulsion for treating cosmetic skin conditions and to a method for producing the oil-in-water emulsion.

The endocannabinoid system (ECS) comprises cannabinoid receptors (CB1 and CB2, TRPV1 and potentially also GPR55), arachidonic acid-derived ligands, and their regulatory enzymes. The importance of the endocannabinoid system in peripheral tissues has been demonstrated in numerous recent studies (Di Marzo V. Targeting the endocannabinoid system: to enhance or reduce? Nat Rev Drug Discov. 2008 May; 7(5):438-55). Whereas the activation of the peripheral endocannabinoid system is often associated with anti-inflammatory and immunosuppressive effects, in the skin the role of the ECS is more complex. CB2 receptor activation has been shown to trigger endorphins that locally act analgesic (Ibrahim MM, Porreca F, Lai J, Albrecht PJ, Rice FL, Khodorova A, Davar G, Makriyannis A, Vanderah TW, Mata HP, Malan TP Jr. CB2 cannabinoid receptor activation produces antinociception by stimulating peripheral release of endogenous opioids. Proc Natl Acad Sci U S A. 2005 Feb 22;102(8):3093-8). Studying CB knockout mice, it has been observed that the ECS and the CB1 receptor can inhibit the pathogenesis of allergic contact dermatitis (Karsak M, Gaffal E, Date R, Wang-Eckhardt L, Rehnelt J, Petrosino S, Starowicz K, Steuder R, Schlicker E, Cravatt B, Mechoulam R, Buettner R, Werner S, Di Marzo V, Tüting T, Zimmer A. Attenuation of allergic contact dermatitis through the endocannabinoid system. Science. 2007 Jun 8;316(5830):1494-7). Interestingly, in this study it was shown that inhibitors of anandamide degradation via fatty acid amide hydrolase (FAAH) could be a promising therapeutic strategy to treat different forms of dermatitis. Overall, the endocannabinoid system in the skin is present, both CB1 and CB2 receptors have been detected in keratinocytes and fibroblasts and the endocannabinoids (EC) are released in the skin where they seem to regulate multiple signals involved in inflammation. Anandamide exerts potent anti-inflammatory and anti-allergic effects (Leonti M, Casu L, Raduner S, Cottiglia F, Floris C, Altmann KH, Gertsch J. Falcarinol is a covalent cannabinoid CB1 receptor antagonists and induces pro-allergic effects in skin. Biochem. Pharmacol. 201 79: 1815-1826). Moreover, anandamide reuptake inhibitors have been shown to exert a number of beneficial effects including neuropathic and peripheral pain (Yates M L, Baker EL. Inactivation and Biotransformation of the endogenous cannabinoids anandamide and 2-arachidonoyl glycerol. Mol. Pharmacol. 2009, 76: 11-17).

WO 2013/174508 A1 describes a new class of compounds, namely 1,3-benzoxazol-2(3H)-ones, as modulators of the endocannabinoid system. The basis for their pharmacological activity is believed to be as follows. The endocannabinoids N-arachidonoylethanolamide (Anandamide, AEA) and 2-arachidonoylglycerol (2-AG) bind to G-protein-coupled CB1 and CB2 cannabinoid receptors. The CB1 receptor is primarily (but not exclusively) expressed in neurons, the CB2 receptor is mainly present in immunocytes, such as monocytes (splenocytes), macrophages, B cells, but also in astroglial cells. In the skin, CB receptors, in particular the CB2 receptor, are expressed in keratinocytes and fibroblasts. Upon CB receptor activation, ECs are actively transported through the cell membrane and degraded. The metabolic enzymes fatty acid amide hydrolase (FAAH) and monoacyl glycerol lipase (MAGL) are responsible for the hydrolysis of AEA and 2-AG, respectively, thus leading to inactivation of these signaling molecules. While activation of the CB1 receptor has been shown to mediate distinct neurophysiological effects, modulation of the CB2 receptor by both agonists and inverse agonists is known to interfere with different inflammatory processes. CB2 receptor activation can lead to anti-inflammatory effects in vivo and CB2 receptor modulation has been implicated in the pathophysiology of a number of diseases, including chronic pain, inflammation of the gastrointestinal tract, osteoporosis, and liver diseases. Plants employ structurally similar lipid signaling molecules as animals to process cellular information, including endocannabinoid-like fatty acid derivatives. Since plants do not generally synthesize arachidonic acid, different N-acylethanolamines like e.g. N-palmitoylethanolamide are produced in plants and these lipids have also been shown to act on proteins directly or indirectly associated with the endocannabinoid system (ECS) in animals and humans, such as FAAH, the transient receptor potential vanilloid receptor (TRPV1) or the newly postulated cannabinoid receptor GPR55.

The 1,3-benzoxazol-2(3H)-ones which are disclosed in WO 2013/174508 A1 show a significant functional interference with the ECS. They either inhibit FAAH or AEA re-uptake. Some compounds of this class even inhibit FAAH in addition to AEA re-uptake. In other words, 1,3-benzoxazol-2(3H)-ones are capable of inhibiting AEA re-uptake or FAAH activity or both without simultaneously effecting CB 1 and CB2 receptors.

However, the class of 1,3-benzoxazol-2(3H)-one compounds may not be formulated into a pharmaceutical/cosmetic cream composition which has a sufficient skin penetration with common formulation techniques. On one hand, it turned out that 1,3-benzoxazol-2(3H)-ones (also termed "active ingredients" or "active pharmaceutical ingredients" or "APIs" in the following) have a very poor solubility in most of the common pharmaceutically/cosmetically acceptable solvents. On the other hand, the inventors have found that 1,3-benzoxazol-2(3H)-ones show a strong tendency for Ostwald ripening in conventional O/W-emulsions. Thus, even in cases in which it has been accomplished to solve a certain amount of the API in a conventional O/W-emulsion, the resulting formulation either proved to be unstable or ineffective in crossing the skin barrier upon topical application. Instability was due to Ostwald ripening of the API-particles which occurred within a few weeks from the preparation of the composition and manifested itself in an unacceptable short shelf-life of the composition and prevented a market launch of such compositions up to now. An ineffective penetration was observed, if less than 10% of the total amount of API have penetrated into excised human skin samples after an application time of 300 minutes at a temperature of 32°C.

In view of the above, it has been an object of the present invention to provide a cream formulation containing an 1,3-benzoxazol-2(3H)-one which is stable and has excellent skin penetration properties. Moreover, it was an aim of the present invention to provide a pharmaceutically/cosmetically effective cream formulation containing sufficient, *i.e.* effective, amounts of 1,3-benzoxazol-2(3H)-one as active ingredient.

These objects have been solved according to the present invention. It was surprisingly found by the inventors that, from the group of 1,3-benzoxazol-2(3H)-ones, the compound N(3-methyl-2-oxo-2,3-dihydrobenzo[d]oxazol-5-yl)dodecanamide is not only pharmaceutically and cosmetically highly effective but can be formulated in a stable and convenient manner at the same time. For instance, in vitro experiments have shown that N-(3-methyl-2-oxo-2,3-dihydrobenzo[d]oxazol-5-yl)dodecanamide is a 1,3-benzoxazol-2(3H)-one which is particularly effective in inhibiting EC uptake. Moreover, it was surprisingly found that this compound can be stably formulated as an oil-in-water emulsion using a specific emulsifier system. In fact, the formulation according to the invention has a low viscosity and an appropriate shelf-life and is suitable for a topical use as medicament and cosmetic, in particular for use as dermatological formulation because it cares and moisturizes the skin.

The invention also comprises pharmaceutically acceptable derivatives, solvates, hydrates and prodrugs of N-(3-methyl-2-oxo-2,3-dihydrobenzo[d]oxazol-5-yl)dodecanamide.

According to the invention, this object is achieved by the provision of the oil-in-water emulsion which comprises 0.01-5.0 wt.-% N-(3-methyl-2-oxo-2,3-dihydrobenzo[d]oxazol-5-yl)dodecanamide or a derivative, solvate, hydrate, or prodrug thereof or a pharmaceutically acceptable salt thereof and further comprises an oil phase, an aqueous phase having a pH in the range of 3.0 to 6.5, an emulsifier system comprising at least one non-ionic polyoxyethylene ether of one or more fatty alcohols, at least one fatty alcohol and at least one glycerol fatty acid ester. Surprisingly, this oil-in-water emulsion has a reduced viscosity which allows for a convenient application of the formulation to the skin. Moreover, the emulsion is improved with respect to its stability. Under inert conditions neither a phase separation of the emulsion into an oil phase and an aqueous phase nor a precipitation of the API occurs at least for a period of 36 months. Further, 99 wt.-% of the API prove to be chemically stable over a period of 36 months. Less than 1 wt.-% of the API degrades during this period when it is in contact with an oxygen containing atmosphere. Apart from that, the formulation has a skin-neutral pH which does not attack the skin's natural coating and simultaneously nourishes the skin. Further surprisingly, the formulation shows an excellent biodistribution: While penetration tests provide evidence that the skin layers absorb high amounts of the API, the blood serum concentration of the API remains low.

According to a first preferred embodiment of the current invention, the aqueous phase comprises at least 92.0 wt.-% of water and at least 3.5 wt.-% of glycerol, more preferably at least 93.0 wt.-% of water and at least 4.0 wt.-% of glycerol. Of course, the water is preferably purified water. If water and glycerol are present in these amounts, it can be ruled out that the aqueous phase contains considerable amounts of other components which could have negative effects on the solubility or stability of the API. In particular, a precipitation or, respectively, crystal growth of the suspended API can be avoided. Moreover, water serves as a solvent to form the aqueous phase of the formulation. Glycerol has a humectant effect and contributes to moisturizing the skin in topical applications of the formulation.

Preferably, the aqueous phase comprises at least 95 wt.%, more preferably at least 97 wt.-%, of a mixture consisting of water, glycerol and at least one preservative agent. The presence of a preservative agent increases the shelf life of the formulation and has an antimicrobial effect. These properties are crucial, if the oil-in-water emulsion is used as a dermatological formulation since the user friendliness scales with the period for which the formulation can be used after opening the container or tube within which the formulation is stored.

The preservative agent is advantageously selected from the group consisting of alkali metal sorbates and alkali metal benzoates. It is even more beneficial to select the preservative agent from the group consisting of potassium sorbate and sodium benzoate. Preferably, the content of potassium sorbate and sodium benzoate when taken together does not exceed 1.5 wt.-% of the emulsion.

The oil-in-water emulsion of the present invention preferably comprises an aqueous phase with a pH of 3.0 to 6.5. More preferably, the pH should fall within the range of 4.5 to 5.5 or within the even smaller range of 4.75 to 5.25. These preferred pH ranges prevent the API from degrading and thus contribute to an increased shelf life of the formulation. In this context it is further beneficial, if the aqueous phase comprises a buffer consisting of citric acid and sodium citrate. The use of this buffer allows keeping the pH of the aqueous phase stable within the predetermined ranges. Alkaline regimes may be avoided, even if a certain amount of an alkaline medium should be added to the formulation.

In another preferred embodiment the O/W emulsion of the present invention contains a smaller oil phase as compared to the water phase. In one embodiment the weight ratio of the aqueous phase to the oil phase is from 2.2 to 2.9, preferably from 2.4 to 2.7. Despite of the relatively small ratio of the oil phase, the emulsion according to this embodiment surprisingly provides for both a better stability (no phase separation) and for improved skin care characteristics.

The weight ratio of the aqueous phase to the emulsifier system may be from 1.6 to 2.2, preferably from 1.7 to 2.1. If the emulsifier system is employed in this ratio, the oil phase is disposed in droplets of appropriate size so that the O/W emulsion is stable over a long period of time.

The emulsion is preferably free from propylene glycol and macrogol 20 glycerol monostearate. Surprisingly, the absence of propylene glycol - which turned out to be a comparatively good solvent for the API - has proved to be beneficial in terms of stability of the emulsion. An undesirable crystal growth (needle formation) of the API is observed when propylene glycol was present. If the emulsion does not contain propylene glycol the formation of the API crystals is considerably deferred.

The emulsifier system may be a combination comprising (or more preferably consisting of) at least one non-ionic polyoxyethylene ether of cetyl and/or stearyl alcohol, at least one fatty alcohol and glycerol monostearate. In this case, the cetyl and/or stearyl alcohol is a co-emulsifier which is added as an emollient and has water absorptive, stiffening and weak emulsifying properties. The non-ionic polyoxyethylene ether is used as a non-ionic emulsifier. The glycerol monostearate is used as a co-emulsifier which has stabilizing properties for the O/W emulsion.

Preferably, the at least one non-ionic polyoxyethylene ether is selected from the group of steareth-2, steareth-21, macrogol cetostearyl ether 12, ceteareth-25, macrogol cetostearyl ether 20 and mixtures of the aforementioned compounds. More preferably, the at least one non-ionic polyoxyethylene ether is selected from the group of ceteareth-25, macrogol cetostearyl ether 20 and mixtures of the aforementioned compounds. In the current composition ceteareth-25, macrogol cetostearyl ether 12 and macrogol cetostearyl ether 20 promote the dispersion of the oil phase in the aqueous of the emulsion. They have ideal dispersion properties due to the lengths of the respective alcohol residues.

More preferably, the non-ionic polyoxyethylene ether is macrogol cetostearyl ether 20. The latter compound can be more easily synthesized or commercially obtained in a pharmaceutical grade than other non-ionic polyoxyethylene ethers, e.g. ceteareth-25.

One preferred example for the emulsifier system is a combination comprising (or more preferably consisting of) macrogol cetostearyl ether 20, cetyl alcohol and glycerol monostearate 40-55. This emulsifier system ensures that the oil phase is dispersed in droplets of appropriate size and that the resulting emulsion has an appropriate viscosity and texture in order to apply it to the skin. It almost goes without saying that the emulsifier system also accounts for an increased stability of the emulsion.

The oil phase comprises or consists of components selected from the group of white soft paraffin, medium-chain triglycerides (MCT) and combinations thereof. Preferably, the oil phase comprises or consists of a mixture of white soft paraffin and MCT. White soft paraffin serves as a lipidic component. MCTs serve as a fat component and emollient. Both oil phase components further improve the penetration behavior of the emulsion and confer stability against oxidation of the API.

The oil-in-water emulsion according to the current invention can include further excipients which are known to the skilled person. These excipients are preferably cosmetically, pharmaceutically and/or dermatologically acceptable compounds. For instance, the further excipients are selected from the group of organic solvents, gelling agents, buffers, detergents, emulsifiers, solubilizers, humectants, fillers, bioadhesives, emollients, preservatives, bactericides, surfactants, perfumes, thickeners, softening agents, moisturizing agents, oils, fats, waxes, water, alcohols, polyols, polymers, foam stabilizers, foaming agents, anti-foaming agents and hair coating agents.

The oil-in-water emulsion according to the current invention may have a composition which comprises or consists of 0.05-2.00 wt.-% N-(3-methyl-2-oxo-2,3-dihydrobenzo[d]oxazol-5-yl)dodecanamide, 60.90-62.85 wt.-% of the aqueous phase, 23.50-25.00 wt.-% oil phase, and 12.10-13.60 wt.-% of the emulsifier system. A weight percentage of 0.05-2.00 wt.-% of the API is sufficient for use as a medicament. The concentration of the API may as well be in a range of 0.05 - 1.20 wt.-%.

The present invention further relates to the above described oil-in-water emulsion for use as medicament, preferably for use as a dermatological formulation. In particular, the emulsion is effective in treating and/or preventing inflammation, irritation, itching, pruritus, pain, oedema, pro-allergic or allergic conditions, alopecia, effluvium, sebaceous gland disorders (e.g. acne, seborrhea), hyperproliferative skin diseases (e.g. psoriasis), excessive hair growth (e.g. hirsutism), dermatitis, in particular atopic dermatitis (AD), dry skin conditions and/or systemic sclerosis (scleroderma). The emulsion is furthermore suitable for topical application onto the skin or mucosa of a mammal, preferably 1-4 times a day, more preferably 1-2 times a day.

Alternatively, the above described oil-in-water emulsion can be used non-therapeutically as a cosmetic, preferably for the cosmetic treatment of the skin and/or mucosa of a mammal. Within the bounds of this use, the emulsion is preferably topically applied onto the skin or mucosa of a mammal, preferably 1-4 times a day, more preferably 1-2 times a day. Additionally, the emulsion may be used non-therapeutically for reducing cosmetic conditions. These include irritation, itching, oedema, pro-allergic or allergic conditions, alopecia, effluvium, sebaceous gland disorders, excessive hair growth and/or dry skin conditions.

According to the present invention, a method for producing the above described oil-in-water emulsion is provided. The method involves combining, in any order, the oil phase (component a), the aqueous phase having a pH in the range of 3.0 to 6.5 (component b), and at least one non-ionic polyoxyethylene ether of one or more fatty alcohols, at least one fatty alcohol and at least one glycerol fatty acid ester (emulsifier system according to component c) with 0.01-5.0 wt.-% of N-(3-methyl-2-oxo-2,3-dihydrobenzo[d]oxazol-5-yl)dodecanamide or a derivative, solvate, hydrate, or prodrug thereof or a pharmaceutically acceptable salt thereof which is micronized prior to the combination with components a)-c) to have a d₉₀ value of at most 100 µm.

In the context of the current invention, the term "micronized" means that the d₉₀ particle size of the API is below a certain threshold value, i.e. 90% of the particles are smaller than a certain threshold value. The d₉₀ particle diameter of the API based on a volume distribution is preferably below a threshold value of 20 µm, more preferably below a threshold value of 18 µm, even more preferably below 15 µm, most preferably below 12 µm. The micronized API can be obtained by grinding larger API particles with suitable equipment. For instance, an appropriate micronization of the API can be obtained by using a jet mill.

In the following the respective compounds of the oil-in-water emulsion of the present invention are described in some more detail:
N-(3-methyl-2-oxo-2,3-dihydrobenzo[d]oxazol-5-yl)dodecanamide N-(3-methyl-2-oxo-2,3-dihydrobenzo[d]oxazol-5-yl)dodecanamide as used herein is a compound according to the following structural formula (I).

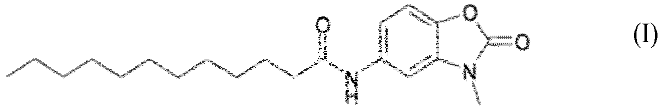

### Derivatives, solvates, hydrates, or prodrugs

The N-(3-methyl-2-oxo-2,3-dihydrobenzo[d]oxazol-5-yl)dodecanamide can be present in the form of a pharmaceutical acceptable derivative, solvate, hydrate or prodrug.

The term "prodrug" as used herein means a derivative of a compound described herein that can hydrolyze, oxidize, or otherwise react under biological conditions (in vitro or in vivo) to provide a compound of the invention. Prodrugs may only become active upon such reaction under biological conditions, or they may have activity in their unreacted forms. Examples of prodrugs contemplated in this invention include, but are not limited to, compounds including a N-(3-methyl-2-oxo-2,3-dihydrobenzo[d]oxazol-5-yl)dodecanamide scaffold and further comprising biohydrolyzable moieties such as biohydrolyzable amides, biohydrolyzable esters, biohydrolyzable carbamates, biohydrolyzable carbonates, biohydrolyzable ureides, and biohydrolyzable phosphate analogues.

The term "derivative" refers to compounds having a N-(3-methyl-2-oxo-2,3-dihydrobenzo[d]oxazol-5-yl)dodecanamide core as a parent compound, but differing from the parent compound in bond order, in the absence or presence of one or more atoms and/or groups of atoms, and combinations thereof. The derivative can differ from the parent compound, for example, in one or more substituents present on the core, which may include one or more atoms, functional groups, or substructures. The derivative can also differ from the parent compound in the bond order between atoms within the core. In general, a derivative can be imagined to be formed, at least theoretically, from the parent compound via chemical and/or physical processes.

N-(3-methyl-2-oxo-2,3-dihydrobenzo[d]oxazol-5-yl)dodecanamide may exist in both unsolvated and solvated forms. The term "solvate" is used herein to describe a molecular association comprising one or more pharmaceutically acceptable solvent molecules, e.g. water or ethanol. The term "hydrate" is used when said solvent is water.

### Macrogol

In the present application the term "macrogol", as common in the pharmaceutical field, is used as a synonym for "polyethylene glycol (PEG)".

### Macrogol cetostearyl ether

In the present application the term "macrogol cetostearyl ethers" refers to compounds which are manufactured by reacting higher saturated fatty alcohols with ethylene oxide. The number in their specific names denotes the degree of ethoxylation (i.e. the average number of repeat units of ethoxy group per molecule). Macrogol cetostearyl ether 20, for example, comprises a hydrophobic part consisting of C₁₆H₃₃- and a hydrophilic part consisting of (OCH₂CH₂)₂₀OH.

### MCT

In the present application the terms "medium-chain triglyceride" and "MCT" refer to a triglyceride with three fatty acids having an aliphatic tail of 6-12 carbon atoms, such as triglycerides of caproic acid, caprylic acid, capric acid and lauric acid.

### Glycerol fatty acid ester

In the present application the term "glycerol fatty acid ester" refers to a glycerol mono fatty acid ester, i.e. a compound composed of a molecule of glycerol linked to a single fatty acid via an ester bond. Examples are glycerol monostearate, glycerol monobehenate, glycerol monocaprylate, glycerol monocaprate and glycerol monolaurate.

### Glycerol monostearate 40-55

Glycerol monostearate 40-55 is a mixture of monoacylglycerols, mainly monostearoylglycerol, together with variable quantities of di- and triacylglycerols. It contains 40.0 per cent to 55.0 per cent of monoacylglycerols, 30.0 per cent to 45.0 per cent of diacylglycerols and 5.0 per cent to 15.0 per cent of triacylglycerols, obtained by partial glycerolysis of vegetable oils mainly containing triacylglycerols of palmitic or stearic acid or by esterification of glycerol with stearic acid 50 (type I), stearic acid 70 (type II) or stearic acid 95 (type III).

The nature of the present invention will become more clearly apparent and better understood from the following examples and accompanying drawings in which:
Figure 1 shows XRPD of N-(3-methyl-2-oxo-2,3-dihydrobenzo[d]oxazol-5-yl)dodecanamide pre (upper curve) and post (lower curve) micronization, first batch.
Figure 2 is a diagram showing plots of three measurements of N-(3-methyl-2-oxo-2,3-dihydrobenzo[d]oxazol-5-yl)dodecanamide pre micronization, first batch.
Figure 3 is a diagram showing plots of three measurements of N-(3-methyl-2-oxo-2,3-dihydrobenzo[d]oxazol-5-yl)dodecanamide post micronization, first batch.
Figure 4 is a graph showing the SEM of N-(3-methyl-2-oxo-2,3-dihydrobenzo[d]oxazol-5-yl)dodecanamide pre micronization, first batch.
Figure 5 is a graph showing the SEM of N-(3-methyl-2-oxo-2,3-dihydrobenzo[d]oxazol-5-yl)dodecanamide post micronization, first batch.
Figure 6 shows XRPD of N-(3-methyl-2-oxo-2,3-dihydrobenzo[d]oxazol-5-yl)dodecanamide pre (upper curve) and post (lower curve) micronization, second batch.

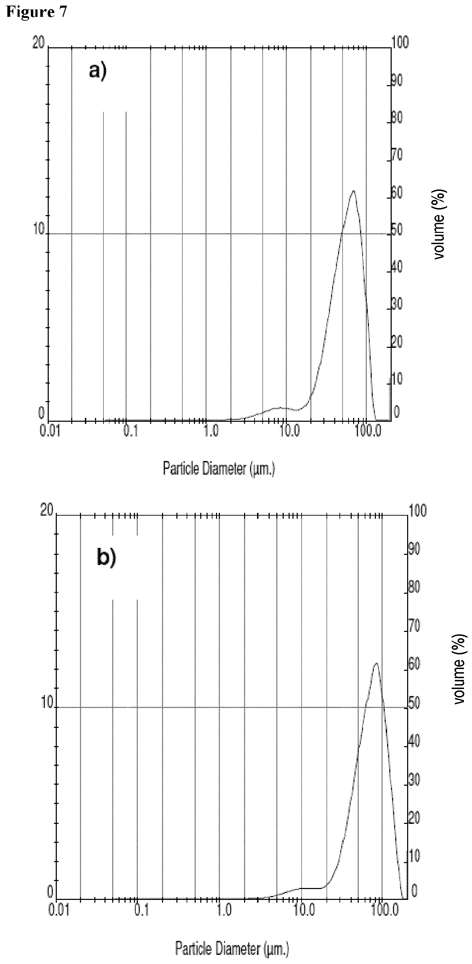

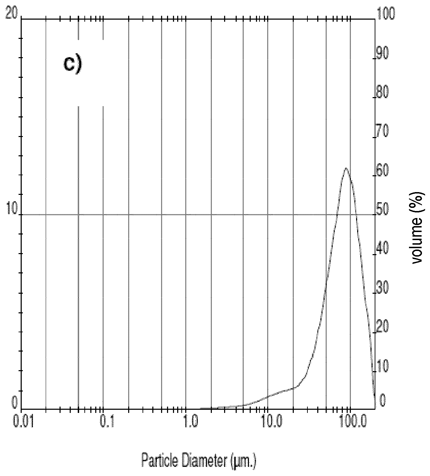

Figure 7 is a diagram showing plots of three measurements of N-(3-methyl-2-oxo-2,3-dihydrobenzo[d]oxazol-5-yl)dodecanamide pre micronization, second batch.

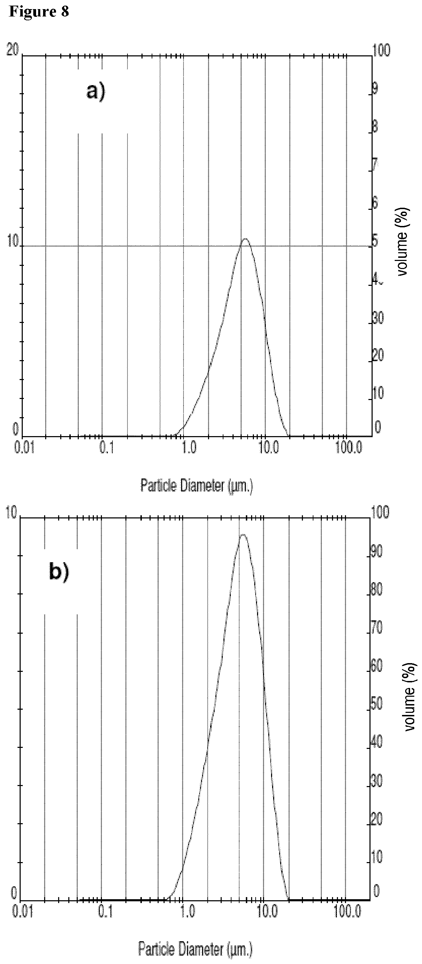

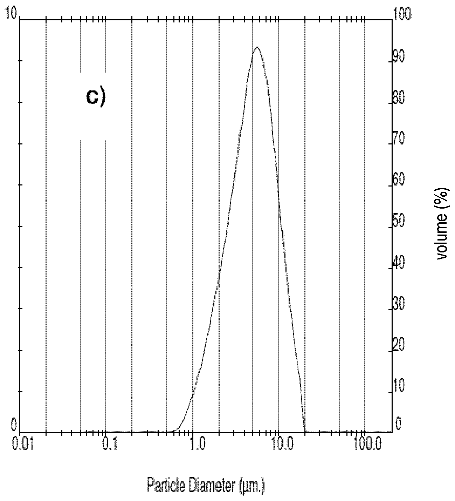

Figure 8 is a diagram showing plots of three measurements of N-(3-methyl-2-oxo-2,3-dihydrobenzo[d]oxazol-5-yl)dodecanamide post micronization, second batch.

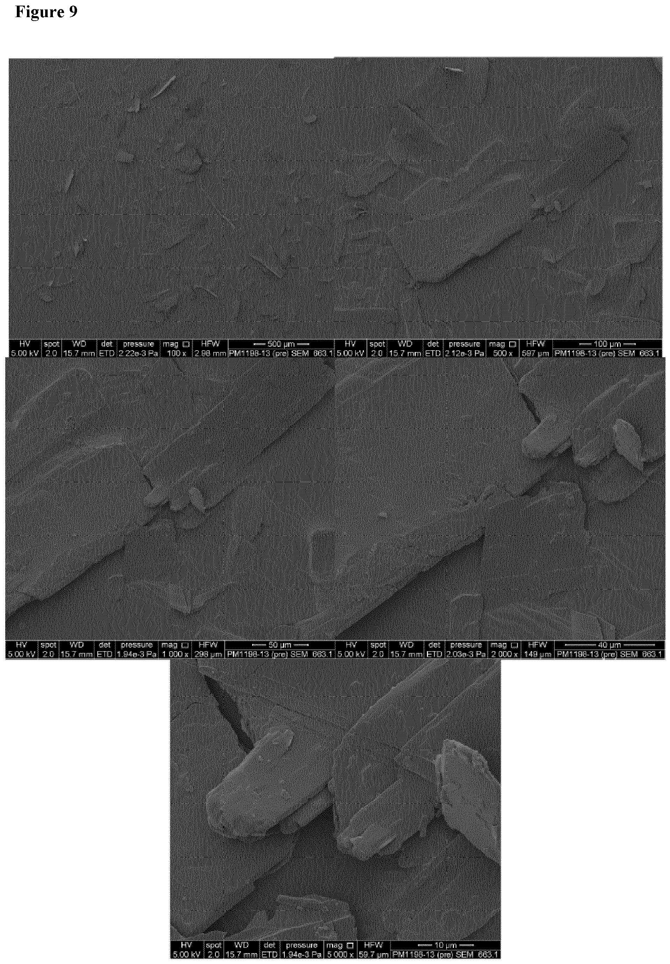

Figure 9 is a graph showing the SEM of N-(3-methyl-2-oxo-2,3-dihydrobenzo[d]oxazol-5-yl)dodecanamide pre micronization, second batch.

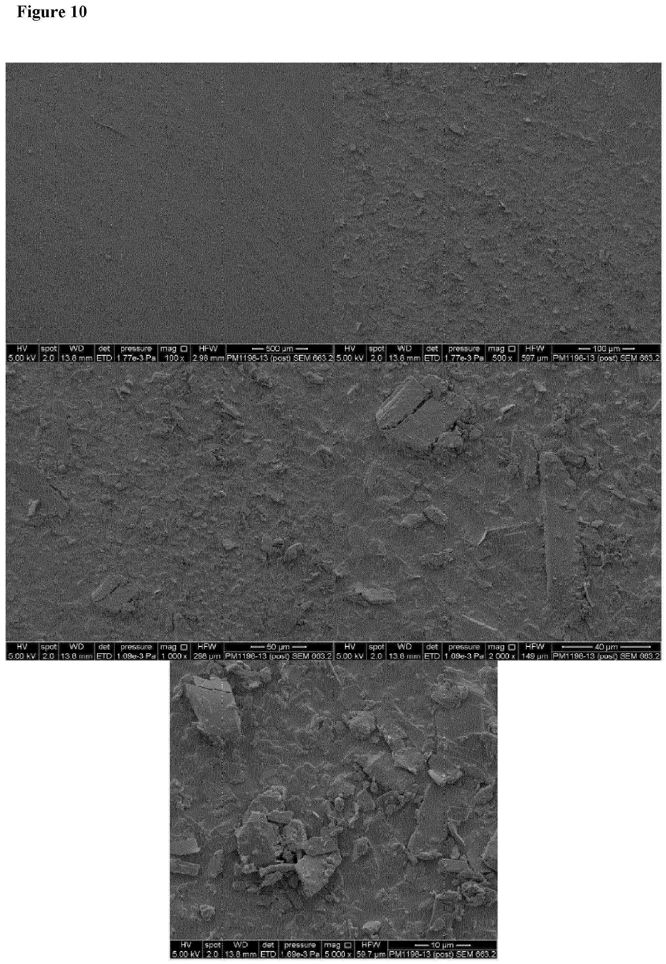

Figure 10 is a graph showing the SEM of N-(3-methyl-2-oxo-2,3-dihydrobenzo[d]oxazol-5-yl)dodecanamide post micronization, second batch.

### Solubility screening

A solubility screening with standard excipients for preparation of a cream base (glycerol monostearate 60, cetyl alcohol, MCTs, paraffin, macrogol-20-glycerol monostearate, propylene glycol and water) was performed.

Simultaneous Thermal analysis (STA = TGA (Thermogravimetric Analysis) + DTA (Differential Thermal Analysis)) was performed in order to test the stability of N-(3 -methyl-2-oxo-2,3-dihydrobenzo[d]oxazol-5-yl)dodecanamide (hereinafter "the API") while heating. Approximately 10 mg of the sample was weighed into a ceramic crucible and placed into the chamber of a Perkin-Elmer STA 600 TGA/DTA analyzer at ambient temperature. The sample was then heated at a rate of 10°C/min from 30°C up to 300°C during which time the change in weight was monitored as well as DTA signal. The purge gas used was nitrogen at a flow rate of 20 cm³/min.

The STA data showed that the material remained stable up to ∼190°C, after which a potential degradation was observed. This meant that the API would not degrade when melting some of the solid excipients.

The following procedure was used to prepare the samples for solubility screen:
1. Weigh out the API
2. Add individual excipient (both solid and liquid excipients)
3. Slurry either by roller at RT if liquid excipient, or by shaker at 60°C if the excipient was solid
4. Shake or sonicate occasionally
5. Check solubility visually, if API not dissolved, add more excipient
6. Repeat steps 3 to 5
7. Samples prepared in steps 1 through 6 were centrifuged and removed from the top for HPLC.
8. Dilute each sample to 1 mL. Only samples fully dissolved were checked by HPLC.

### Results:

Based on the solubility screen it was concluded that the API showed the following solubility (according to general notices, Ph.Eur.):

| | |
|---|---|
| MCTs: | Very slightly soluble |
| Propylene glycol: | Very slightly soluble |
| Water: | Practically insoluble |

In the general notice of Ph.Eur. in statements of solubility the terms used have the following significance, referred to at a temperature between 15°C and 25°C.

| Descriptive term | Approximate volume of solvent in milliliters per gram of solute | | | |
|---|---|---|---|---|
| Very slightly soluble | from | 1,000 | to | 10,000 |
| Practically insoluble | more than | | | 10,000 |

### Micronization by jet mill

A Sturtevant qualification micronizer was used to reduce the particle size of the API. The mill was operated with tangential flow (i.e. the nitrogen supply and drug are fed in the same direction in the milling chamber). The material of API as received was fed into the jet mill using a Venturi feed system, where air was used to draw the feed material into the milling chamber. A product filter bag was affixed to the outlet of the mill, through which the exhausts and the milled drug substance collects. The milling conditions were set as follows: Grind gas: Dry nitrogen gas; Grind pressure: 80 psi; Feed pressure: 80 psi; Room conditions: Ambient.

### First Batch (Laboratory Scale - 5 g)

The yield (or recovery rate) of the first trial of micronization in which a 5 g sample of N-(3-methyl-2-oxo-2,3-dihydrobenzo[d]oxazol-5-yl)dodecanamide was treated by jet mill was better than 85%.

### Second Batch (Laboratory Scale - 200 g)

In a second experiment an upscaling to ∼200 g was performed. N-(3-methyl-2-oxo-2,3-dihydrobenzo[d]oxazol-5-yl)dodecanamide was micronized by jet mill using the conditions described above. The yield (or recovery rate) of the micronization by jet mill was better than 75%.

### X-Ray Powder Diffraction (XRPD)

Samples were analyzed using a PANalytical X'pert PRO XRPD. The equipment was checked as with a standard operating procedure (SOP) using a silicon disk and resulting data fell within the necessary specifications. Approximately 5-30 mg of sample was placed into a zero background XRPD disk sample holder. The sample was then loaded into the instrument and diffraction patterns recorded using the following experimental conditions: Tube anode: Cu; Start angle [2 theta]: 5.0084; Generator tension: 40 kV; End angle [2 theta]: 49.9904; Generator current: 40 mA; Time per step: 31.0097 seconds; Wavelength alpha1: 1.5406 Å; Step size [2 theta]: 0.0170; Wavelength alpha2: 1.5444 Å

### First Batch

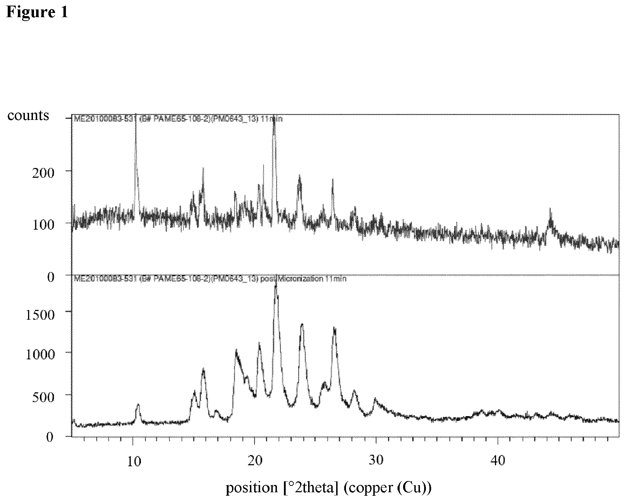

Figure 1 shows the XRPD trace of N-(3-methyl-2-oxo-2,3-dihydrobenzo[d]oxazol-5-yl)dodecanamide pre (upper curve) and post (lower curve) micronization. There is no specific difference in diffraction peaks (i.e. the position) observed, suggesting the material is predominantly the same physical form post micronization. Apparently, the FWHM (full width at half maximum) of each diffraction peak was all largely increased, indicating that the particle diameter (grain size) of the crystals was significantly reduced post micronization.

### Second Batch

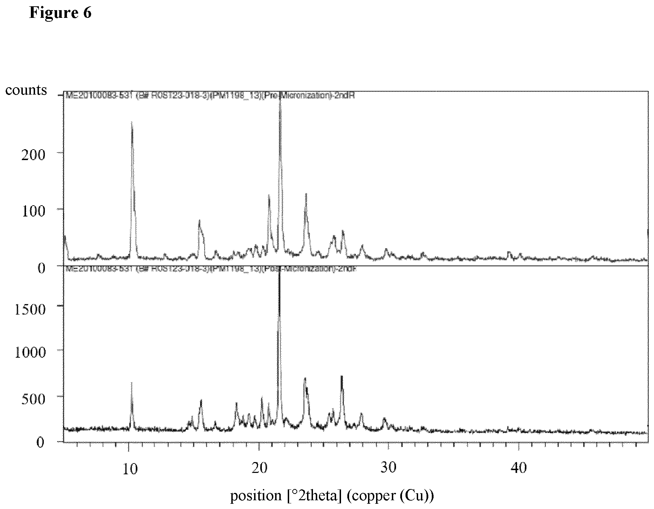

Figure 6 shows the XRPD trace of N-(3-methyl-2-oxo-2,3-dihydrobenzo[d]oxazol-5-yl)dodecanamide pre (upper curve) and post (lower curve) micronization. There is no specific difference in diffraction peaks (i.e. the position) observed, suggesting the material is predominantly in the same physical form post micronization.

### Particle size determination (PSD) of N-(3-methyl-2-oxo-2,3-dihydrobenzo[d]oxazol-5-yl)dodecanamide

A representative sample, dispersed at an adequate concentration in a suitable liquid, is passed through the beam of a monochromatic light source, preferably from a laser. The light scattered by the particles at various angles is measured by a multi-element detector, and numerical values relating to the scattering pattern are then recorded for subsequent analysis. These numerical scattering values are then transformed, using an appropriate optical model and mathematical procedure, to yield the proportion of total volume to a discrete number of size classes forming a volumetric particle size distribution (e.g., dso describes a particle diameter corresponding to 50% of the cumulative undersize distribution, i.e. 50% of the particles have a diameter that is smaller than a threshold value).

The Particle Size Distribution was determined using Malvern Mastersizer MicroPlus particle size analyzer (Malvern Instruments, UK) and 1% (v/v) Tween 80 in deionized water was used as dispersant for this experiment. The equipment was turned on and the following conditions were set: Analysis Model: Polydisperse; Presentation Code: Frauenhofer (5$$D); Stirring speed: 10 o'clock dial position.

The equipment was left to warm up for about 1 hour and approximately 100 mL of the dispersant was added to the sample dispersion unit. Backgrounds were first measured using the dispersant. A fresh sample was prepared by adding -20 to 50 mg API into ∼2 to 3 mL of the dispersant and this was sonicated for ∼5 minutes. The sample was added dropwise into the sample dispersion unit while stirring the dispersant until a suitable obscuration value was achieved and the particle size distribution could be measured. A minimum of three measurements were made for the pre and post micronized samples.

Alternatively, the Malvern Mastersizer 2000 can be used for the determination of Particle Size Distribution. In this case water is used as dispersant.

### First Batch

The particles size was reduced post micronization by jet mill, e.g. d₉₀ was reduced from 106.06±56.69 µm (mean ± SD) for the starting material to 6.76±0.36 µm post micronization (cf. table 1). A more uniform PSD was also obtained post micronization as seen in the much smaller SD than that of the staring material in three measurements.

**Table 1:**

| **Sample** | **Micronization** | **d₁₀ (µm)** | **d₅₀ (µm)** | **d₉₀ (µm)** |
|---|---|---|---|---|
| **N-(3-methyl-2-oxo-2,3-dihydrobenzo[d]oxazol-5-yl)dodecanamide** | pre | 7.53±1.55 | 33.71±18.61 | 106.06±56.69 |
| | post | 1.11±0.05 | 2.97±0.05 | 6.76±0.36 |

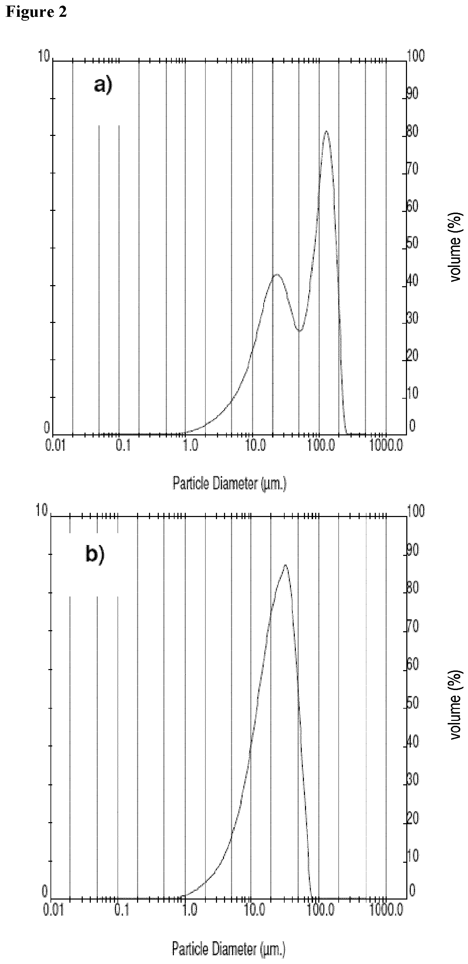

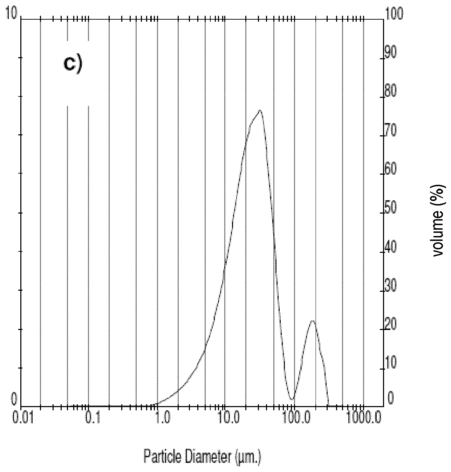

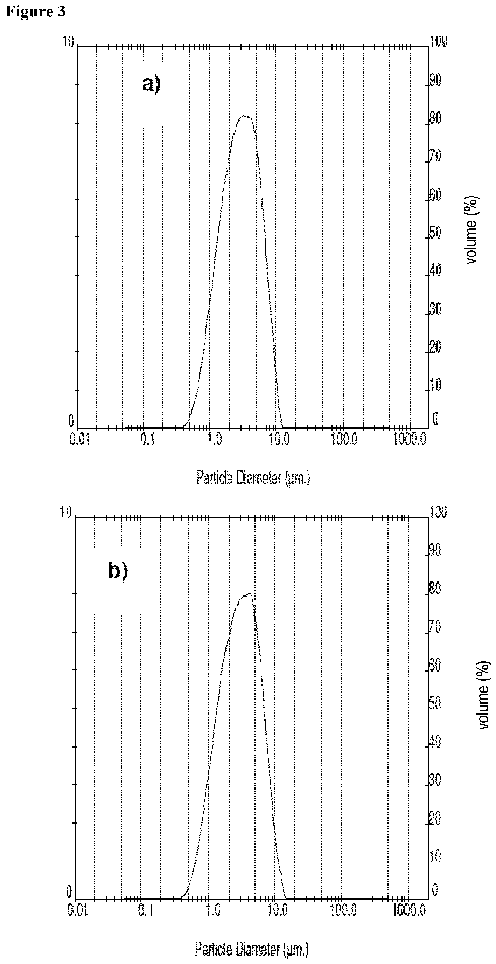

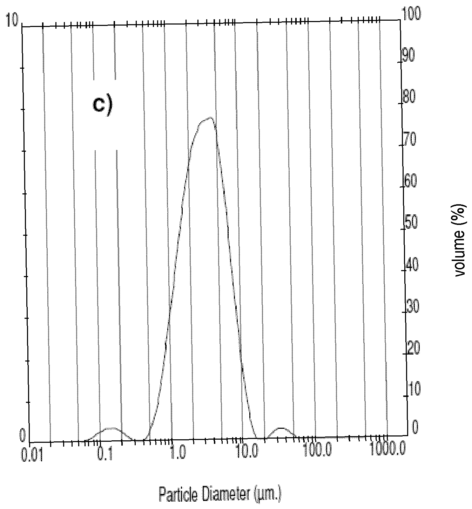

Plots of three measurements of particle size distribution pre and post micronization are shown in Figures 2 and 3.

### Second Batch

The particles size was reduced post micronization by jet mill, e.g. d₉₀ was reduced from 127.67±18.59 µm (Mean ± SD) for the starting material to 10.29±0.44 µm post micronization (cf. table 2). More uniform PSD was also obtained post micronization as seen in the much smaller SD than that of the staring material in three measurements.

**Table 2:**

| **Sample** | **Micronization** | **d₁₀ (µm)** | **d₅₀ (µm)** | **d₉₀ (µm)** |
|---|---|---|---|---|
| **N-(3-methyl-2-oxo-2,3-dihydrobenzo[d]oxazol-5-yl)dodecanamide** | pre | 26.90±3.65 | 69.71±9.95 | 127.67±18.59 |
| | post | 1.96±0.11 | 4.98±0.10 | 10.29±0.44 |

### SEM (Scanning Electron Microscope)

Scanning Electron Micrographs were produced by coating a sample of N-(3-methyl-2-oxo-2,3-dihydrobenzo[d]oxazol-5-yl)dodecanamide with a thin layer of gold (sputter coating) and examining it using a FEI-Philips XL30 scanning electron microscope. The acceleration voltage of the electrons used for the analysis was 5.0 KV. All images were captured with a computer-controlled CCD camera attachment.

### Results obtained for the pre-micronization state of the sample (Batch 1)

Morphology: Large thin sheets;
Size: Large plane but very thin, non-uniform
Agglomeration: Large thin sheets can be packed together

### Results obtained for the post-micronization state of the sample (Batch 1)

Morphology: Mostly irregular particles with a few small/medium sheets
Size: Smaller, more uniform
Agglomeration: Some thin sheets can also be packed together

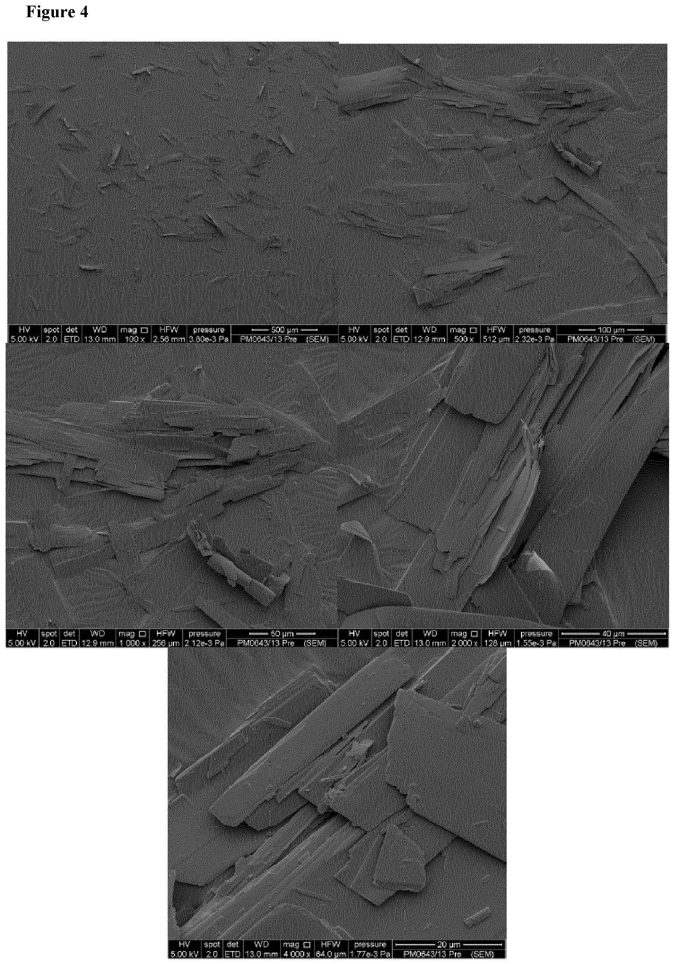

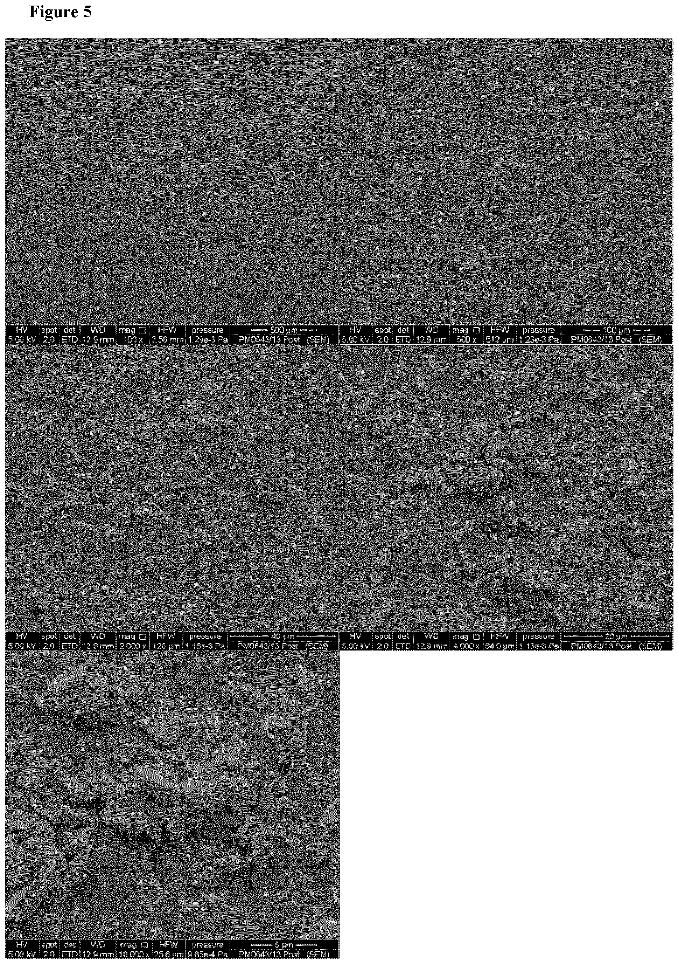

In summary, the particle size was largely reduced by micronization and with more uniform distribution. The SEM pre and post micronization are shown in Figures 4 and 5.

### Summary micronization and characterization

### First Batch

Physical characterization of N-(3-methyl-2-oxo-2,3-dihydrobenzo[d]oxazol-5-yl)dodecanamide pre and post micronization by jet mill (scale: ∼5 g) was carried out using X-ray powder diffraction (XRPD), scanning electron microscope (SEM) and particle size distribution (PSD). XRPD and SEM data indicated that the compound has no changes in physical form post micronization. PSD data showed that the particle size could be reduced from over 100 µm to below 10 µm (d₉₀) with more uniform distribution post micronization. A good yield (or recovery rate) was also obtained in micronization of N-(3-methyl-2-oxo-2,3-dihydrobenzo[d]oxazol-5-yl)dodecanamide.

### Second Batch

Physical characterisation of N-(3-methyl-2-oxo-2,3-dihydrobenzo[d]oxazol-5-yl)dodecanamide pre and post micronization (scale ∼200 g) was carried out using X-ray powder diffraction (XRPD), SEM (not shown) and PSD. XRPD and SEM data indicated that the compound has no changes in physical form post micronization. PSD data showed that the particle size could be reduced from over 100 µm to about 10 µm (d₉₀) with more uniform distribution post micronization. A good yield (or recovery rate) was also obtained at ∼75% in micronization of this compound at a scale of 200 g.

### Upscaling

For upscaling purposes, the micronization process was transferred to a jet mill AS200 (Hosokawa Alpine, Germany) on which the following experiments were performed. Dry nitrogen and air were used as grind gas in order to determine the gas which is more suitable for the micronization process.

### Further Batches (150 and 200 g)

Two further batches of N-(3-methyl-2-oxo-2,3-dihydrobenzo[d]oxazol-5-yl)dodecanamide (150 and 200 g, resp.) were micronized with the requirement d₉₀ ≤10 µm. The 150 g batch was micronized using normal air whereas the 200 g batch was micronized with dry nitrogen as grind gas. The micronized product was obtained in 79% (d₉₀ = 7.43 µm) and 78% (d₉₀ = 8.92 µm) yield.

Physical characterization of the two further batches was carried out using X-ray powder diffraction (XRPD) and scanning electron microscope (SEM) applying the methods which have been described above. Based on the characterization, the following was concluded: XRPD data indicated that the diffraction pattern of the further two batches correspond favorably to that of the first and the second batch. Further, there are no significant differences between the further two batches and the first and the second batch as observed by SEM. Last not least, the results show that the micronization process can also been performed using the jet mill AS200. Moreover, it was shown that it is not required to use dry nitrogen as grind gas.

### Additional Batches (kg scale)

Four additional batches were micronized using the jet mill AS200. The results are shown in table 3.

**Table 3:**

| | | | | |
|---|---|---|---|---|
| **Batch size (kg)** | **1.035** | **0.913** | **1.073** | **3.60** |
| **d₉₀ (µm)** | 5.5 | 4.8 | 4.4 | 7.6 |
| **XRPD** | complies | complies | complies | complies |

The results shown in table 3 indicate that the micronization process can be upscaled.

### Forced degradation studies

Solid samples and sample solutions of N-(3-methyl-2-oxo-2,3-dihydrobenzo[d]oxazol-5-yl)dodecanamide were exposed to stress agents for a defined period and influence of stress on the compound were studied (i.e. the degradation).

The evaluation was expressed as relative percent with respect to t=0 result of unstressed sample (mass balance, % of peak areas).

The stress conditions were adjusted in such way that the degradation varied in a range of approx. 1 to 5% generated impurities.

The following stress conditions were applied: acidic and basic hydrolysis, oxidizing agent, temperature and photolytic conditions.

### Photolytic conditions (VIS, UV)

Solid sample - daylight, laboratory condition (VIS) - exposure time 7 days
Solid sample - UV 254 nm, laboratory condition (UV) - exposure time 6 days

### Temperature conditions (T)

Solid sample, 70°C - exposure time 6 days

### Oxidizing conditions (H₂O₂)

Sample solution, 3% H₂O₂, laboratory condition - exposure time 24 h

### Acidic hydrolysis (AH)

Sample solution, 0.1M HCI, 50°C - exposure time 24 h

### Basic hydrolysis (BH)

Sample solution, 0.01-0.05M NaOH, 25°C (1M NaOH, 50°C, 1 h) - exposure time 45 min

### Summary of forced degradation results

### Photolytic conditions (VIS, UV)

The UV-VIS forced degradation study showed good stability of the stressed sample. Mass balances after these tests were 100.2 and 102.4%. Impurity profiles of reference preparation and stressed sample preparation are very similar.

It can be concluded that the API is photo stable.

### Temperature conditions (T)

Elevated temperature storage of the sample did not cause the formation of degradation products. Mass balance after this test was 102.6%. Impurity profiles of reference preparation and stressed sample preparation are very similar.

It can be concluded that the API is thermo stable.

### Oxidizing conditions (H₂O₂)

The stressed sample was stable for at least 24 hours when using 3% H₂O₂ solution. Oxidizing conditions resulted in no changes (decreasing) in the content of the API in the stressed sample. This is also illustrated by the mass balance, which is 98%.

Impurity profile of the DMSO diluted sample shows 5 unknown impurities in ranges of 0.05-0.09% of peak area (reference preparation) and 5 unknown impurities in range 0.07-0.14% of peak area in the stressed sample. There are no significant changes in the impurity profile during the 24 hours testing.

It can be concluded that the API is stable under an oxidizing condition.

### Acidic hydrolysis (AH)

The acidic hydrolysis condition generated a stability indicating impurity. Mass balance after this test was 101.2%.

It can be concluded that the API is stable in an acidic condition.

### Basic hydrolysis (BH)

Basic hydrolysis testing demonstrated high instability of the sample in this condition. After 1 hour the sample was completely degraded by 1M NaOH).

From this it can be concluded that the sample is very unstable in a strongly alkaline condition using an enhanced temperature of the sample during exposure. For the next testing 0.01M and 0.05M NaOH was used.

Basic hydrolysis condition (0.05M NaOH, 5 min, room temperature) generated 5 degradation impurities. Purity of the measured sample was 89.50% A. Levels of the generated impurities are in range from 0.09 to 4.13%A.

The API is not stable in alkaline conditions.

### Feasibility Study

Compositions 1 and 2 have been prepared in order to investigate whether human skin is capable of absorbing N-(3-methyl-2-oxo-2,3-dihydrobenzo[d]oxazol-5-yl)dodecanamide at all. To that end, the API (component 6) has been mixed with a ready-to-use DAC cream having the indicated ingredients (components 1-5 and 7-8). A conventional mixer (e.g. Ultra Turrax, Becomix RW 50, RW 60, and RW 320) has been used.

### Composition 1:

| **Position** | **Substance** | **g/100 g** |
|---|---|---|
| **1** | Glycerol monostearate 60 | 3.96 |
| **2** | Cetyl alcohol | 5.94 |
| **3** | MCTs | 7.43 |
| **4** | Macrogol-20 glycerol monostearate type II | 6.93 |
| **5** | Paraffin, white soft | 25.25 |
| **6** | N-(3 -methyl-2-oxo-2,3-dihydrobenzo[d]oxazol-5-yl)dodecanamide, non-micronized | 1.00 |
| **7** | Propylene glycol | 9.90 |
| **8** | Purified water | 39.60 |

### Composition 2:

| **Position** | **Substance** | **g/100 g** |
|---|---|---|
| **1** | Glycerol monostearate 60 | 3.96 |
| **2** | Cetyl alcohol | 5.94 |
| **3** | MCTs | 7.43 |
| **4** | Macrogol-20 glycerol monostearate type II | 6.93 |
| **5** | Paraffin, white soft | 25.25 |
| **6** | N-(3 -methyl-2-oxo-2,3-dihydrobenzo[d]oxazol-5-yl)dodecanamide, micronized | 1.00 |
| **7** | Propylene glycol | 9.90 |
| **8** | Purified water | 39.60 |

Once prepared, Compositions 1 and 2 have been tested in a skin penetration study (see below). However, the compositions have not been checked for their stability or homogeneity.

### First Set of Formulations

The first set of formulations has been prepared by combining the ingredients indicated below using a conventional mixer (e.g. Ultra Turrax, Becomix RW 50, RW 60, and RW 320). All ingredients are commercially available. Glycerol monostearate 40-55 is also known as glycerol monostearate 40-55% (Type II).

### Comparative Formulation 1:

| **Position** | **Substance** | **g/100 g** |
|---|---|---|
| **1** | Glycerol monostearate 40-55 | 3.00 |
| **2** | Cetyl alcohol | 4.50 |
| **3** | MCTs | 5.60 |
| **4** | Macrogol-20 glycerol monostearate type II | 5.25 |
| **5** | Paraffin, white soft | 18.75 |
| **6** | Benzyl alcohol | 1.00 |
| **7** | N-(3 -methyl-2-oxo-2,3-dihydrobenzo[d]oxazol-5-yl)dodecanamide, micronized | 0.30 |
| **8** | Propylene glycol | 10.00 |
| **9** | Purified water | 51.60 |

### Comparative Formulation 2:

| **Position** | **Substance** | **g/100 g** |
|---|---|---|
| **1** | Glycerol monostearate 40-55 | 3.00 |
| **2** | Cetyl alcohol | 4.50 |
| **3** | MCTs | 5.60 |
| **4** | Macrogol-20 glycerol monostearate type II | 5.25 |
| **5** | Paraffin, white soft | 18.75 |
| **6** | Benzyl alcohol | 1.00 |
| 7 | N-(3 -methyl-2-oxo-2,3-dihydrobenzo[d]oxazol-5-yl)dodecanamide, micronized | 1.00 |
| **8** | Propylene glycol | 10.00 |
| **9** | Purified water | 50.90 |

A suspension of N-(3-methyl-2-oxo-2,3-dihydrobenzo[d]oxazol-5-yl)dodecanamide was obtained in Comparative Formulations 1 and 2.

The formulations as described in Comparative Formulations 1 and 2 were tested in a 28-day dermal toxicity study in Göttingen minipigs (see below). Moreover, Comparative Formulations 1 and 2 were subjected to stability testing for 12 months. The results showed that the formulations were physically stable up to 12 months. The chemical stability of the active pharmaceutical ingredient (API) was also confirmed. However, starting from month 6, crystal growth of the API (needle formation) was observed.

### Second Set of Formulations

The subsequent experiments focused on the development of a set of formulations in which N-(3-methyl-2-oxo-2,3-dihydrobenzo[d]oxazol-5-yl)dodecanamide was dissolved in the long term. Therefore, the solubility of N-(3-methyl-2-oxo-2,3-dihydrobenzo[d]oxazol-5-yl)dodecanamide has been tested in several excipients and hexylene glycol has been identified as the most promising candidate. Hence, the subsequent experiments have been conducted with 10% hexylene glycol and 0.1% N-(3-methyl-2-oxo-2,3-dihydrobenzo[d]oxazol-5-yl)dodecanamide.

These formulations proved to be physically unstable and had a consistency which was not acceptable for further development. Additionally, these formulations where physically unstable or had an unacceptable consistency and showed crystal formation of the API.

### Third Set of Formulations

The next set of experiments focused on incorporating N-(3-methyl-2-oxo-2,3-dihydrobenzo[d]oxazol-5-yl)dodecanamide as a suspension into the formulations. An acid pH value was chosen for the aqueous phase of these formulations.

### Comparative Formulation 3:

| **Position** | **Substance** | **g/100 g** |
|---|---|---|
| **1** | N-(3 -methyl-2-oxo-2,3-dihydrobenzo[d]oxazol-5-yl)dodecanamide, micronized | 1.00 |
| **2** | Glycerol monostearate 40-50 | 3.00 |
| **3** | Cetylalcohol | 4.50 |
| **4** | MCTs | 5.60 |
| **5** | PEG-20 glyceryl stearate | 5.25 |
| **6** | Paraffin, white soft | 18.75 |
| **7** | Benzylalcohol | 1.00 |
| **8** | Propylene glycol | 10.00 |
| **9** | Citric acid, anhydrous | 0.25 |
| **10** | Sodium citrate | 0.35 |
| **11** | Purified water | 50.30 |

The formulation of Comparative Formulation 3 was physically stable. However, a crystal growth of the API was observed.

### Formulation Example 1:

| **Position** | **Substance** | **g/100 g** |
|---|---|---|
| **1** | Glycerol monostearate 40-55 | 3.00 |
| **2** | Cetyl alcohol | 4.50 |
| **3** | MCTs | 5.60 |
| **4** | Macrogol Cetostearyl Ether 20 | 5.25 |
| **5** | Paraffin, white soft | 18.75 |
| **6** | N-(3 -methyl-2-oxo-2,3-dihydrobenzo[d]oxazol-5-yl)dodecanamide, micronized | 1.00 |
| **7** | Potassium sorbate | 0.20 |
| **8** | Sodium benzoate | 0.10 |
| **9** | Citric acid, anhydrous | 0.25 |
| **10** | Sodium citrate | 0.35 |
| **11** | Glycerol (85%) | 3.00 |
| **12** | Purified water | 58.00 |

### Formulation Example 2:

| **Position** | **Substance** | **g/100 g** |
|---|---|---|
| **1** | Glycerol monostearate 40-55 | 3.00 |
| **2** | Cetyl alcohol | 4.50 |
| **3** | MCTs | 5.60 |
| **4** | Macrogol Cetostearyl Ether 20 | 5.25 |
| **5** | Paraffin, white soft | 18.75 |
| **6** | N-(3 -methyl-2-oxo-2,3-dihydrobenzo[d]oxazol-5-yl)dodecanamide, micronized | 0.30 |
| **7** | Potassium sorbate | 0.20 |
| **8** | Sodium benzoate | 0.10 |
| **9** | Citric acid, anhydrous | 0.25 |
| **10** | Sodium citrate | 0.35 |
| **11** | Glycerol (85%) | 3.00 |
| **12** | Purified water | 58.70 |

### Formulation Example 3:

| **Position** | **Substance** | **g/100 g** |
|---|---|---|
| **1** | Glycerol monostearate 40-55 | 3.00 |
| **2** | Cetyl alcohol | 4.50 |
| **3** | MCTs | 5.60 |
| **4** | Macrogol Cetostearyl Ether 20 | 5.25 |
| **5** | Paraffin, white soft | 18.75 |
| **6** | N-(3 -methyl-2-oxo-2,3-dihydrobenzo[d]oxazol-5-yl)dodecanamide, micronized | 0.10 |
| **7** | Potassium sorbate | 0.20 |
| **8** | Sodium benzoate | 0.10 |
| **9** | Citric acid, anhydrous | 0.25 |
| **10** | Sodium citrate | 0.35 |
| **11** | Glycerol (85%) | 3.00 |
| **12** | Purified water | 58.90 |

### Stability studies

Formulation Examples 1-3 exhibited a good stability of the O/W emulsions over 36 months during stability testing (stability of emulsion in terms of phase separation and in terms of stability of the API). In an in-use stability test containers that had been opened were analyzed again after 12 months. The good stability was confirmed also under these conditions.

### Particle size determination (formulation)

Microscopic examination (particle size measurement) was performed on Formulation Examples 1-3 in the course of the stability studies. Two batches of each formulation were stored up to 36 months at 25 °C/60 % RH and 30 °C/75 % RH as well as for 6 months at 40 °C/75 % RH. Ostwald ripening was not observed throughout the observation period. Exemplary particle size data determined from microscopic images of the samples which have been stored for 36 months in total are shown in Table 4 after intervals of 12, 24 and 36 months.

**Table 4:**

| **Determination of particle size:** | | | **recorded results** | |
|---|---|---|---|---|
| | **Initial** | **12 m** | **24 m** | **36 m** |
| | 100 %: < 100 µm | < 85 µm | < 55 µm | < 100 µm |

Initially, all particles were smaller than 100 µm, i.e. the biggest particle observed had a size of 100 µm. After 36 months no change to the initial particle size could be noticed. The microscopic image after 36 months has, overall, been inconspicuous.

### Skin Penetration studies

Skin penetration studies were performed with excised human skin. Tissue samples were washed with saline postoperatively and the subcutaneous fat layer was removed. Punch biopsies (20 mm diameter, 3.14 cm²) were taken and stored at -20°C. At the beginning of the penetration study the full thickness skin sample was thawed and dried with a swab. Penetration studies were performed applying Franz diffusion cells. The formulations or compositions containing N-(3-methyl-2-oxo-2,3-dihydrobenzo[d]oxazol-5-yl)dodecan-amide were applied on the skin and distributed equally. The skin sample on gaze was placed on the diffusion cell which was tempered at 32°C before. After 30, 100 and 300 min, respectively, remaining formulation or composition was removed with a swab. After removal from the diffusion cell three punch biopsies (6 mm diameter) were taken. Horizontal sections were prepared from which the API was extracted. N-(3-methyl-2-oxo-2,3-dihydrobenzo[d]oxazol-5-yl)dodecanamide content in all extracts and the acceptor medium was analyzed with HPLC-MS.

In the penetration study performed with Composition 1 most of the applied N-(3-methyl-2-oxo-2,3-dihydrobenzo[d]oxazol-5-yl)dodecanamide was found in the swab (88-96% of the applied dose). Only 6-8% of the API penetrated into the skin (6% after an application time of 30 min, 8% after an application time of 100 min, 7% after an application time of 300 min).

Micronization of N-(3-methyl-2-oxo-2,3-dihydrobenzo[d]oxazol-5-yl)dodecanamide had a positive effect on the penetration into skin. In the study performed with Composition 2, 17-28% of the API penetrated into the skin (28% after an application time of 30 min, 19% after an application time of 100 min, 17% after an application time of 300 min) and only 39-56% of the initially applied amount were found in the swabs.

In a penetration study performed with Formulation Example 1, 15-25% of the applied N-(3-methyl-2-oxo-2,3-dihydrobenzo[d]oxazol-5-yl)dodecanamide penetrated into skin (21% after an application time of 30 min, 15% after an application time of 100 min, 25% after an application time of 300 min) whereas 40-44% of the applied amount were found in the swab.

### Toxicokinetics

A 28-day dermal toxicity study was performed in which Comparative Formulations 1 and 2 were applied on a daily basis (2 mg/cm², ∼480 cm²). Toxicokinetic measurements were performed on day 28. The Cₘₐₓ was determined to be 23-62 ng/mL.

In a 7-day dermal local tolerance and toxicity study Formulation Example 1 was applied on a daily basis (2 mg/cm², ∼480 cm²). Toxicokinetic measurements were performed on day 7. The Cₘₐₓ was determined to be 48.7 pg/mL.

### Biological assays

In order to prove the therapeutic effect of N-(3-methyl-2-oxo-2,3-dihydrobenzo[d]oxazol-5-yl)dodecanamide (denoted "test compound" in the following) different biological assays have been carried out.

### A. Radioligand Displacement Assays on CB₁ and CB₂ Receptors

For the CB1 receptor, binding experiments were performed in the presence of 0.5 nM of a radioligand [³H]CP55,940 (168 Ci/mmol) (Perkin Elmer, Waltham, MA, US) at 30°C in siliconized glass vials together with 8.0 µg of membrane recombinantly over-expressing CB1 (No. RBHCB1M, Perkin Elmer, Waltham, MA, US), which was resuspended in 0.5 mL (final volume) assay-buffer (50 mM TRIS-HCl, 2.5 mM EDTA, 5 mM MgCl₂, 0.5 mg/mL fatty acid free BSA, pH 7.4). The test compound was present at varying concentrations and the non-specific binding of the radioligand was determined in the presence of 10 µM WIN55,212-2 (Tocris Cookson Ltd., Bristol, UK). After 2 h incubation, the suspension was rapidly filtered through 0.1% polyethylenimine pre-soaked UniFilter^{®}-96 GF/B (Perkin Elmer, Boston MA, US) washed twelve times with 167 µL ice-cold assay-buffer. Radioactivity on dried and sealed filter plates was measured with a Perkin Elmer 1450 Microbeta TRILUX liquid scintillation counter in 40 µL MicroScint 20 scintillation cocktail (Perkin Elmer, Waltham, MA, US). Data collected from three independent experiments performed in triplicates were normalized between 100% and 0% specific binding for [³H]CP55,940. These data were graphically linearized by projecting Hill-plots, which allowed the calculation of IC₅₀ values. Derived from the dissociation constant (K*_{D}*) of [³H]CP55,940 and the concentration-dependent displacement (IC₅₀ value), inhibition constants (*Kᵢ*) of competitor compounds were calculated using the Cheng-Prusoff equation [*Kᵢ* = IC₅₀/(1 + L/K_{D})].

For CB2 receptor binding studies 3.8 µg of membrane recombinantly over-expressing CB2 (No. RBXCB2M, Perkin Elmer, Waltham, MA, US) were resuspended in 0.5 mL assay buffer (see above) together with 0.5 nM of the radioligand [³H]CP55,940. The CB2-binding assay was conducted in the same manner as for CB1.

### B. Critical micelle concentration (CMC) measurements

Dye micellization was carried out with a modified version of the method described by Eliyahu et al., Novel dextran-spermine conjugates as transfecting agents: comparing watersoluble and micellar polymers. Gene Ther. 2005 Mar;12(6):494-503. The test compound (from a 2 mM stock solution) was mixed at increasing concentrations with 0.1 nM fluorescein (free acid, 99%, Fluka, Switzerland) at room temperature in Nanopure distilled water. Experiments were carried out on 96-well microtiter plates (excitation at 485 nm, emission at 535 nm). Since the emission of fluorescein is pH-dependent, the pH of the mixture was kept constant at 6.9. The CMC range was determined as the concentration range where the first statistically significant increase in fluorescence was detected. At an appropriate resolution of the concentration range this increase was not gradual but sudden.

### C. Cellular uptake of [³H]-AEA

Human lymphoma U937 cells were grown in RPMI 1640 medium (Invitrogen, Basel, Switzerland) supplemented with 10% fetal bovine serum, 1 g/mL fungizone (amphotericin B), 100 units/mL penicillin, 100 g/mL streptomycin, and 2 mM L-glutamine (all from Invitrogen) and selected for the cellular AEA uptake assay. 2.5 x 10⁶ cells were collected and suspended in 250 µL of RPMI 1640 medium (Invitrogen, Basel, Switzerland) at 37°C using glass silanized vials. Cells were pre-incubated 15 min at 37°C in presence of the test compound in a concentration range of 10⁻¹¹ - 10⁻⁵ M or in presence of the same amount (5 µL) of vehicle control (DMSO). Then a mixture of 0.5 nM [³H]-AEA (60 Ci/mmol) (American Radiolabeled Chemicals, Inc., San Louis, MO, US) and 99.5 nM of nonradioactive AEA (Tocris Cookson Ltd., Bristol, UK) resulting in a final concentration of 100 nM AEA was added and samples were incubated at 37°C for another 15 minutes. The reaction was stopped by rapid filtration over UniFilter^{®}-96 GF/C (Perkin Elmer, Boston MA, US) pre-soaked with PBS 0.25% BSA (w/v). Cells were washed three times with ice-cold PBS buffer containing 1% fatty acid free BSA (137 mM NaCl, 2.7 mM KCl, 10 mM Na₂HPO₄, 1.76 mM KH₂PO₄, 1% BSA fatty acid free, pH 7.4), dried and 40 µL MicroScint 20 scintillation cocktail (Perkin Elmer, Waltham, MA, US) was added. Radioactivity was measured using a Perkin Elmer 1450 Microbeta TRILUX liquid scintillation counter. Results were expressed as % of AEA uptake versus cells treated with solvent and the EC₅₀ and IC₅₀ values were calculated from the sigmoidal curves, which were generated using the GraphPad Prism 5.0 (GraphPad Software, San Diego, CA, US).

For the test compound an EC₅₀ of 4.0 nM has been calculated.

### D. Fatty acid amide hydrolase (FAAH) inhibition

FAAH activity was assessed using pig brain or U937 homogenate according to the method described by Omeir et. al. Arachidonoyl ethanolamide-[1,2-14C] as a substrate for anandamide amidase. Life sciences 56, 1999-2005 (1995) and adapted by Fowler et al. Selective inhibition of anandamide cellular uptake versus enzymatic hydrolysis--a difficult issue to handle. European journal of pharmacology 492, 1-11 (2004).

Briefly, 10 µL of vehicle or positive control URB597 (Cayman Chemical, Ann Arbor, MI, US) resulting a final concentration of 100 nM or of the inhibitor at the adequate concentrations were pre-incubated at 37°C for 15 min at 450 rpm with 490 µL of diluted pig brain (200 µg per sample) or U937 homogenate (0.63 mg protein per sample corr. 10⁶ U937 cells) in FAAH activity buffer (10 mM Tris-HCl and 1 mM EDTA, pH 8.0, 0.1% w/v fatty acid free BSA (Sigma, San Louis, MO, US)). A mixture of 0.5 nM [³H]AEA (60 Ci/mmol) and 99.5 nM of nonradioactive AEA resulting in a total concentration of 100 nM AEA was added and incubated for 15 min. Successively, 1 mL of a mixture 1:1 (v/v) of methanol:chloroform was added to each sample and, after vigorous vortexing, aqueous and organic phases were separated by centrifugation at 10'000 rpm for 10 min at 4°C. The radioactivity associated to the [³H]-ethanolamine (produced by FAAH-catalyzed breakdown of [³H]-AEA) was measured upon addition of 3 mL of Ultima Gold scintillation liquid (Perkin Elmer, Waltham, MA, US) to the aqueous (upper) phase, using a PACKARD TRI-CARB 2100TR Liquid Scintillation Analyzer. Results were expressed as % of FAAH activity vs. vehicle treated homogenate. Collected data was normalized by subtraction of unspecific signal at total inhibition of FAAH by URB597. Single values of FAAH inhibition or IC₅₀ values from generated sigmoidal curves were calculated using GraphPad Prism 5.0 (GraphPad Software, San Diego, CA, US).

### E. Monoacylglycerol lipase (MAGL) inhibition

MAGL activity was evaluated using the Monoacylglycerol Lipase Inhibitor Screening Assay Kit (Cayman Chemicals, MI, US) and the experimental procedure was carried out according to the kit protocol. Briefly, 10 µL of the test compound at different concentrations (range 0.1-20 µM) were added to 150 µL of assay buffer, 10 µL of human recombinant MAGL and 10 µl of arachidonoyl-1-thio-glycerol (150 µM in the assay). The plate was incubated at room temperature for 5 min, afterward 10 µL of DTNB (5, 5'-dithiobis-(2-nitrobenzoic acid), Ellman's reagent) was added to each well. The plate was shaken for 10 seconds and the absorbance measured at 415 nm using Genius Pro spectrofluorimeter (Tecan, Grodig, Austria). Initial 100% enzymatic activity was calculated using solvent instead of the test compound, whilst background was measured without the human recombinant enzyme. The blank was subtracted from each value and results were expressed as % of initial enzymatic activity.

### F. In vivo model for pruritus associated with the oxazolone model of a delayed type hypersensitivity reaction

Scratching activity in mice is measured after topical application of the test compound. Ear thickness is measured and histology parameters are determined (see e.g. Elliott G.R. An automated method for registering and quantifying scratching activity in mice: use for drug evaluation. J. Pharmacol. Toxicol. Methods. 2000;44:453-459 and Gijbels M.J. Therapeutic interventions in mice with chronic proliferative dermatitis (cpdm/cpdm). Exp. Dermatol. 2000;9:351-358).

While the invention has been described and illustrated in reference to certain preferred embodiments thereof, those skilled in the art will appreciate that various changes, modifications and substitutions can be made therein without departing from the spirit and the scope of the invention. It is intended that the invention be limited only by the scope of the claims which follow and that such claims be interpreted as broadly as is reasonable.

## Claims

1. An oil-in-water emulsion containing 0.01-5.0 wt.-% N-(3-methyl-2-oxo-2,3-dihydrobenzo[d]oxazol-5-yl)dodecanamide or a derivative, solvate, hydrate, or prodrug thereof or a pharmaceutically acceptable salt thereof and further comprising
(a) an oil phase;
(b) an aqueous phase having a pH in the range of 3.0 to 6.5; and
(c) an emulsifier system comprising
at least one non-ionic polyoxyethylene ether of one or more fatty alcohols,
at least one fatty alcohol and
at least one glycerol fatty acid ester.

2. The oil-in-water emulsion according to claim 1, wherein the aqueous phase comprises at least 92.0 wt.-% of water and at least 3.5 wt.-% of glycerol, more preferably at least 93.0 wt.-% of water and at least 4.0 wt.-% of glycerol.

3. The oil-in-water emulsion according to claim 1, wherein the aqueous phase comprises at least 95 wt.%, preferably at least 97 wt.-%, of a mixture consisting of water, glycerol and at least one preservative agent.

4. The oil-in-water emulsion according to one of the preceding claims, wherein the aqueous phase comprises at least one preservative agent selected from the group consisting of alkali metal sorbates and alkali metal benzoates, preferably from the group consisting of potassium sorbate and sodium benzoate.

5. The oil-in-water emulsion according to one of the preceding claims, wherein the aqueous phase comprises a buffer consisting of citric acid and sodium citrate and/or the aqueous phase has a pH from 4.5 to 5.5, preferably from 4.75 to 5.25.

6. The oil-in-water emulsion according to one of the preceding claims, wherein the weight ratio (b)/(a) is from 2.2 to 2.9, preferably from 2.4 to 2.7, and the weight ratio (a)/(c) is from 1.6 to 2.2, preferably from 1.7 to 2.1.

7. The oil-in-water emulsion according to one of the preceding claims, wherein the emulsion is free from propylene glycol and macrogol 20 glycerol monostearate.

8. The oil-in-water emulsion according to one of the preceding claims comprising as the emulsifier system a combination comprising or consisting of at least one non-ionic polyoxyethylene ether of cetyl and/or stearyl alcohol,
at least one fatty alcohol and
glycerol monostearate.

9. The oil-in-water emulsion according to one of the preceding claims comprising as the emulsifier system a combination comprising or consisting of macrogol cetostearyl ether 20,
cetyl alcohol and
glycerol monostearate 40-55.

10. The oil-in-water emulsion according to one of the preceding claims, wherein the emulsion comprises or consists of
0.05-2.00 wt.-% N-(3-methyl-2-oxo-2,3-dihydrobenzo[d]oxazol-5-yl)dodecanamide, 60.90-62.85 wt.-% of the aqueous phase,
23.50-25.00 wt.-% oil phase, and
12.10-13.60 wt.-% of the emulsifier system.

11. The oil-in-water emulsion according to one of the preceding claims for use as medicament, preferably as dermatological formulation.

12. The oil-in-water emulsion according to one of the preceding claims for use in treating and/or preventing inflammation, irritation, itching, pruritus, pain, oedema, pro-allergic or allergic conditions, alopecia, effluvium, sebaceous gland disorders, hyperproliferative skin diseases, excessive hair growth, dermatitis, in particular atopic dermatitis (AD), atopic eczema, dry skin conditions and/or systemic sclerosis.

13. Non-therapeutic use of the oil-in-water emulsion according to claims 1-10 as a cosmetic, preferably for the cosmetic treatment of the skin and/or mucosa of a mammal.

14. The oil-in-water emulsion for use according to one of claims 11 or 12 or the non-therapeutic use of the oil-in-water emulsion according to claim 13, wherein the emulsion is topically applied onto the skin or mucosa of a mammal, preferably 1-4 times a day, more preferably 1-2 times a day.

15. Method for producing the oil-in-water emulsion according to one of the preceding claims by combining, in any order,
0.01-5.0 wt.-% of N-(3-methyl-2-oxo-2,3-dihydrobenzo[d]oxazol-5-yl)dodecanamide or a derivative, solvate, hydrate, or prodrug thereof or a pharmaceutically acceptable salt thereof, which is micronized to have a d₉₀ value of at most 100 µm, with
(a) the oil phase,
(b) the aqueous phase having a pH in the range of 3.0 to 6.5, and
(c) at least one non-ionic polyoxyethylene ether of one or more fatty alcohols, at least one fatty alcohol, and at least one glycerol fatty acid ester.
